# EUROPEAN PATENT SPECIFICATION

(11) **EP 4 175 694 B1**
(45) Date of publication and mention of the grant of the patent: **01.05.2024**
(21) Application number: 21739560.7
(22) Date of filing: 02.07.2021
(51) Int. Cl.: A61M 3/02

(54) **AN IRRIGATION SYSTEM**
BEWÄSSERUNGSSYSTEM
SYSTÈME D'IRRIGATION

(30) Priority: 03.07.2020 DK PA202070457; 04.12.2020 DK PA202070812
(43) Date of publication of application: 10.05.2023
(73) Proprietor: Coloplast A/S, 3050 Humlebaek (DK)
(72) Inventor: HICKMOTT, Richard Morgan, 3000 Helsingoer (DK); WANSCH, Aaron, 21153 Malmoe (SE)
(86) International application number: PCT/DK2021/050224
(87) International publication number: WO 2022/002340

(56) References cited:
- US-A- 5 807 320
- US-A1- 2011 306 941
- US-A1- 2020 206 411
- US-B1- 6 669 668

## Description

The invention relates to a system for anal irrigation and a box for use in a system for anal irrigation.

### Brief Description of the Drawing

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Figure 1 illustrates an anal irrigation system positioned on a thigh of a user and ready for use.
Figure 2 illustrates a reservoir, tube and anal probe in one disposable element.
Figure 3 illustrates a perspective view of an anal irrigation system with a reservoir, tube and anal probe and a box including means for pressing.
Figure 4 illustrates a one-way foil valve suitable to be used in a reservoir.
Figure 5 illustrates a cross-sectional view of an anal irrigation system with a reservoir inserted into a cavity of a box and where the reservoir is subjected to press from means for pressing in the box.
Figures 6A, 6B illustrate cross-sectional views of an anal irrigation system with a reservoir inserted into a cavity of a box. Figure 6A illustrates the system prior to use and figure 6B illustrates the system during use.
Figures 7A, 7B illustrate cross-sectional views of an anal irrigation system with a reservoir inserted into a cavity of a box. Figure 7A illustrates the system prior to use and figure 6B illustrates the system during use.
Figures 8A, 8B illustrate cross-sectional views of an anal irrigation system with a reservoir inserted into a cavity of a box. Figure 8A illustrates the system prior to use and figure 8B illustrates the system during use.
Figures 9A, 9B illustrate a top view and a cross-sectional view of an anal irrigation system including a box with two chambers. Figure 9A illustrates a top view of the box with an open lid and figure 9B illustrates a cross-sectional view with the box with a closed lid.
Figure 10 illustrates a cross-sectional views of an anal irrigation system with a reservoir inserted into a cavity of a box.
Figures 11 and 12 illustrate reservoirs.
Figures 13 and 14 illustrate perspective views of an anal irrigation system with roller functioning as means for pressing.
Figures 15 to 17 illustrate a box with foldable arms and how it can be positioned during use.
Figures 18 to 23 illustrate steps of preparing a box and a reservoir for use in an anal irrigation system.
Figure 24 illustrate a cross-sectional view of a box for use in anal irrigation system.
Figures 25-27 illustrate, in an embodiment not forming part of the invention as claimed, a box with an inflatable lung, which can be used without a reservoir

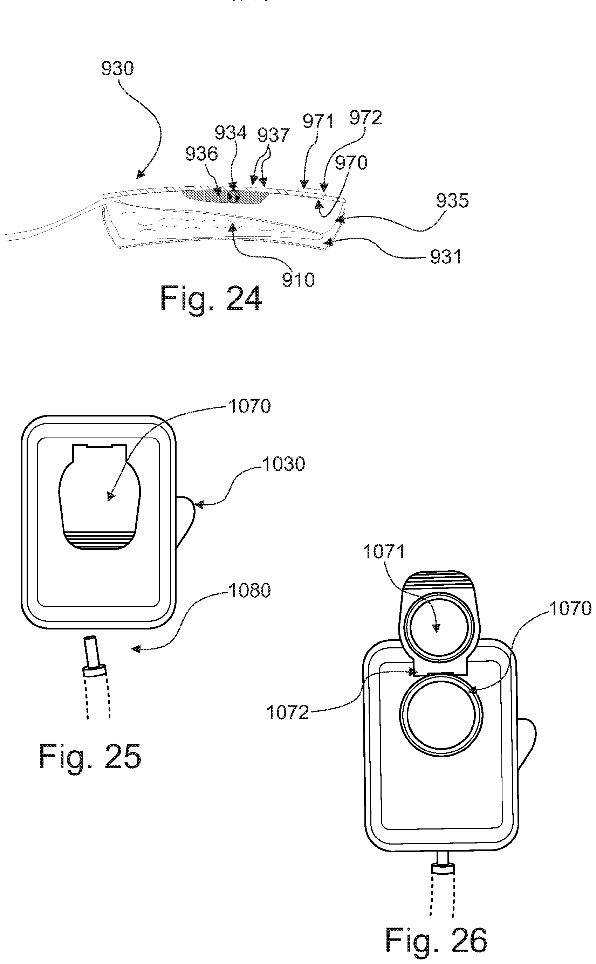

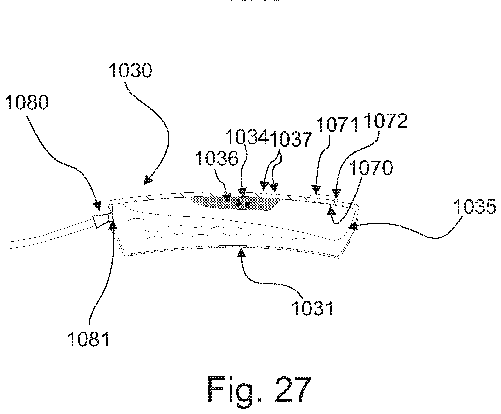

Figures 25-26 illustrate top views and figure 27 illustrate a cross-sectional view.

### Detailed Description

Trans-anal irrigation, or rectal irrigation, is referred to as anal irrigation and provides a process for emptying the lower bowel of faecal matter. Anal irrigation benefits people who suffer from neurological disorders associated with a spinal injury, spina bifida, multiple sclerosis, Parkinson's disease, or other neurological diseases; sensory disorders that might arise after surgery to treat colitis; or issues of tissue elasticity associated with aging. People who benefit from anal irrigation often have limited dexterity.

Relevant prior art is for instance disclosed in documents US5807320 A, US2020/206411 A1, US6669668 B1 and US2011/306941 A1.

An anal irrigation system according to the present invention comprises the technical features as defined in independent claim 1.

Embodiments described below provide an anal irrigation system (AIS) having a disposable component and a reusable component. The disposable component includes in some embodiments a reservoir to hold an amount of liquid, a probe for insertion into the rectum, and a tube connected between the reservoir and the probe. In other embodiments not forming part of the invention as claimed, the disposable component includes a reservoir to hold an amount of liquid, the reservoir being connectable to a reusable tube, which in turn is connectable to a disposable probe. The reusable component provides a box or a container that receives the reservoir of the disposable component, where the box is adapted to compress the reservoir to displace the liquid from the reservoir and out of the probe.

The advantages of the AIS include a compact box that can be easily handled by the user, where the box is simple in form and is not pressurized. The box provides a variety of mechanical approaches for reducing a space inside of the box, which proportionally collapses the walls of the reservoir to displace a proportional amount of liquid out of the reservoir and through the anal probe. The box generally has smooth, clean exterior walls that allow the user to easily handle the box. The internals of the box move or otherwise displace to reduce an effective inside volume of the box. The reduced internal volume of the box results in the reservoir being squeezed to displace the liquid in the reservoir.

During use, the reservoir is inserted into the box with the tube and anal probe projecting out of the box. The user initiates a compression of the reservoir, where for example, the effective internal space of the box is reduced (by walls coming together or by a roller motion), which pushes the liquid out of the reservoir and through the anal probe. The reservoir, tube, and probe are in some embodiments discarded upon completion of the anal irrigation. In other embodiments not forming part of the invention as claimed, the tube may be reused, and the reservoir and probe are discarded upon completion of the anal irrigation. The box is not pressurized, or in other words, the box is not a pressure vessel. Thus, the liquid flowing from the probe can be selectively and wilfully stopped by the user without moving valves or depressurization of the system pressure. The user has direct and better control and receive the amount of irrigation they desire. There is no need to clean the reservoir or probe between uses as they are discarded.

Embodiments according to the invention as claimed relate to an anal irrigation system comprising:
- an anal probe, a reservoir configured to contain irrigation liquid and tubes connecting the reservoir with the anal probe, the reservoir having flexible wall portions and
- a box having a shell that forms an exterior surface of the box and defines an interior space within the box, and the interior space of the box includes a reservoir holding cavity,
- the box having means for pressing on an exterior surface of the reservoir thereby displacing the irrigation liquid from the reservoir.

The anal probe is firmly and not detachably connected to the tube, which in turn is firmly and not detachably connected to the reservoir.

Users of anal irrigation systems typically have poor hand dexterity. Therefore, simple systems requiring little, or no setup are advantageous for the users. Systems as described in this disclosure are very simple to set up prior to use and requires no, or almost no connections to be made prior to use.

The box as disclosed herein provides an easy and convenient way for displacing irrigation liquid from a reservoir. This is an advantage, in particular for users with poor hand dexterity, who might find it difficult to use an irrigation system.

In the context of this disclosure the proximal end of the anal probe, the tube and so forth is the end closest to the user in use and the distal end is the opposite end.

The anal probe may be in the form of a through-going tubular element with an interior lumen configured for allowing flow of irrigation liquid. The anal probe will typically include retention means. The outer diameter of such an anal probe is typically about 8-16 mm, for example 10 mm. The length is about 70-200 mm, for example about 150 mm.

The box described in this disclosure may be made of rigid material such as Acrylonitrile butadiene styrene (ABS), Polycarbonate (PC), combinations of PC and ABS, acrylic (PMMA), Polypropylene PP or other types of plastic material.

The box includes any of several means for pressing as further described below. The material of the box has a rigidity such that it is able to withstand the pressure from the means for pressing and to provide a backstop for the pressure. However, the box is not a pressure vessel and need not be pressurised to deliver irrigation liquid to the probe, i.e., the box is not gas-tightly sealed.

In case no reservoir is used, the box is liquid tight. This means that the box is provided with sealings at any opening into the box. As mentioned above, any provision of sealing is not configured for providing a gas-tight seal.

In the context of this disclosure, the means for pressing configured for displacing liquid means that the liquid is displaced directly by the means and without pressure being built-up in the box. The anal irrigation system including the box does not rely upon valves or stops to meter or contain an increased pressure in the box for release at a later stage. Contrary to this, the system is configured to have a direct link between the pressing by the means for pressing and the displacement of liquid. In other words, the means for pressing are configured to displace the liquid by direct interaction between the means for pressing and the reservoir, in such a way that activation of the means for pressing leads to liquid being displaced.

The reservoir is made of flexible material or at least comprising flexible wall portions configured for be compressed by the means for pressing. Examples of material are Polyethylene, Polyvinylchloride or Thermoplastic elastomeric material. Environmentally friendly material such as corn starch may also be used. The reservoir may be made of a very thin-walled material with a thickness of less than 100 µm or even in the range of 10 to 50 µm. The reservoir is configured for containing approximately 1 litre of irrigation liquid but may be larger or smaller, if preferred. The irrigation liquid may be tap water but may also include a saline solution.

Embodiments relate to the means for pressing to be a roller configured for curling up the reservoir. The roller may include a slot so that the reservoir is inserted into the slot, caught here and curled up around the roller to thereby displace the liquid from the reservoir. In these embodiments, the box may have a scraper on a sidewall behind the roller, so as to assist in pushing out the liquid from the reservoir.

Embodiments relate to means for pressing in the form of an actuator in combination with a pressure plate. The linear actuator may be electrically driven so that the user can manipulate it by simply pressing a button on the box.

By using a pressure plate, it is possible to easily and with certainty check how far the pressure plate is moved. This provides an accurate reading of how much the volume of reservoir has been decreased and thereby how much liquid has been irrigated through the system and thus instilled into the bowels of the user.

The actuator may be positioned at a sidewall of the box and be configured for pushing the pressure plate towards an opposite sidewall. In these cases, the actuator is a linear actuator.

The pressure plate may be connected by a hinge to a sidewall of the box and configured for being tilted. In these cases, the actuator may be rotatable or linear or both.

Embodiments relate to the means for pressing to be in the form of an inflatable lung.

The inflatable lung may be inflated by an air pump. The air pump may be electrically or manually driven. The inflatable lung may be subdivided into chambers, such as two, three, four or five chambers. The subdivision will allow the user to detect how much liquid has been irrigated into the bowels, because each chamber may be able to displace a certain amount of liquid from the reservoir.

Embodiments relate to the box including a lid.

Embodiments relate to the lid including the means for pressing.

Embodiments relate to the anal probe being firmly and not detachably connected to the tube, which in turn is firmly and not detachably connected to the reservoir.

This provides for a very simple and reliable system. Prior to use, the user fills liquid into the reservoir and inserts the reservoir into the box and then the system is ready to use. No further preparation or connection of the system is needed. Following use, the user simply removes the reservoir including the tube and anal probe from the box and disposes these single-use components. No disconnection of tubes, cleaning of tubes and so forth is required. Furthermore, because the system is connected when the user receives it, such a system removes any possibility of user-induced faulty connections which might lead to misuse of the system.

Embodiments relate to the anal probe including a foam element on an exterior surface of the tubular element of the anal probe, which foam element is configured for retaining the anal probe in the rectum during the irrigation procedure. The foam element may be in the form of a cuff.

Embodiments relate to the anal probe including an inflatable balloon.

In these embodiments, the box may include a second line for pumping air into the inflatable balloon or a second chamber.

Embodiments relate to the box comprising holding means configured for holding the box in place at the upper side of the thighs of a user. The holding means may be configured for holding the box in place at an upper side of one thigh or may alternatively be configures for holding the box in place at an upper side of both thighs.

Embodiments relate to the box comprising a strap configured for holding the box in place at a thigh of a user. The strap may be made of a suitable fabric and comprise hooks and loops for closing it. As an example, the strap may be made of polychloroprene.

Embodiments relate to the anal irrigation system or the box comprising a pair of foldable arms, which are configured for holding the box in place at a thigh of a user.

The distance between the arms may be adapted for allowing positioning over one thigh or may alternatively be adapted for allowing positioning over both thighs of a user.

The arms may be made of the same material as the box and may extend 20-25 cm downwards from a lower side of the box in an unfolded configuration. Transverse to the folding direction, the arms may be 10 cm wide. To allow for the folding/un-folding of the arms, they may each be provided with a hinge extending transversely across the arm in a distance of 2-5 cm from the lower side of the box. The hinge may be a living hinge or a friction hinge. The distance between the arms may be 10-20 cm, or larger depending on whether the distance is adapted for fitting the arms over one thigh or both thighs. In case, the arms are adapted for fitting over both thighs, the distance between them may be 30 cm or more. The arms may also be configured for being folded outwards (180 degrees) so as to provide a larger base for the box. In this way, the box with the completely unfolded arms may be positioned across both thighs.

Alternatively, the arms may be configured for being positioned in a variety of positions. The arms may have a multitude of hinged sections so that they are malleable in various positions.

Embodiments relate to the reservoir being provided with first coupling means and the box being provided with second coupling means, wherein the first coupling means are configured for cooperating with the second coupling means so as to provide a coupling for filling the reservoir when it is situated in the box.

Such embodiments are advantageous in case the reservoir is thin-walled and very flexible. A thin-walled and very flexible reservoir will be somewhat flimsy in structure and may therefore be difficult to control during filling and subsequent insertion into the box. Therefore, it may be an advantage to couple the reservoir to the box prior to filling and then fill the reservoir through the box. For that purpose, the box is provided with a filling opening positioned at the coupling.

Embodiments relate to the first coupling means being 50-100 mm in diameter. The second coupling means may also be 50-100 mm in diameter. In embodiments the first coupling means comprise a c-shaped channel configured for cooperating with a ridge on the second coupling means so as to provide a coupling between the first and second coupling means. In embodiments, the first coupling means comprise a first upstanding ridge, which is configured for cooperating with a second upstanding ridge on the second coupling means, such that the first upstanding ridge circumferentially surrounds the second upstanding ridge. The first upstanding ridge may in this embodiment be rolled over the second upstanding ridge for coupling the two coupling means together.

Embodiments relate to the box having a closure for closing the filling opening. The closure may be hinged to a top wall of the box.

Embodiments relate to the tubes being connected to the box through a connector on the tube configured for cooperating with a through-going opening at the box. These embodiments are particularly useful, if the anal irrigation system has no reservoir, because the box in this case is closed and liquid tight. However, connection of the tubes to the box may also be done in embodiments provided with a reservoir.

The connector may as examples be a luer-lock type of connector, a threaded connector or a bayonet connector.

### Detailed Description of the Drawing

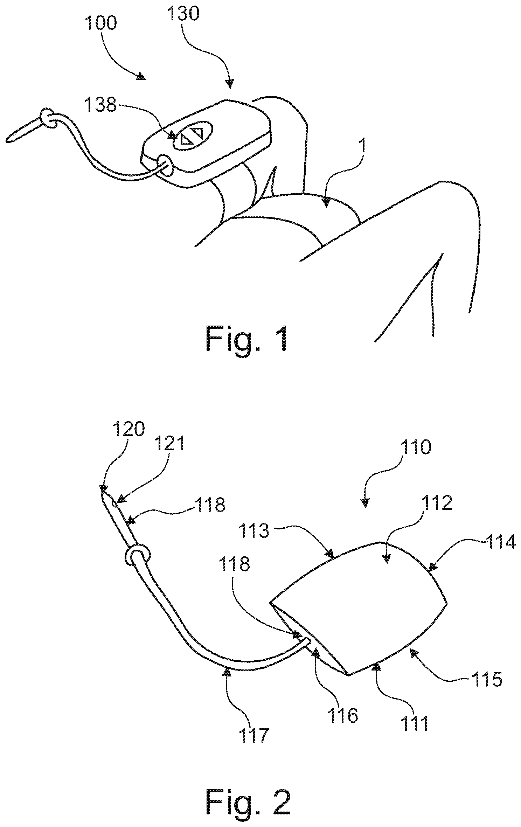

Figures 1 and 2 illustrate sketches of an irrigation system 100 as disclosed herein. The irrigation system 100 is in figure 1 shown in a ready-to-use state positioned on a user who is sitting on a toilet-seat 1 (only partly shown). The system comprises a box 130 configured for holding a reservoir 110 (Figure 2). The reservoir 110 in the illustrated embodiment is configured for containing approximately 1 litre of irrigation liquid, but may be larger or smaller, if desired. The box 130 is made of rigid material as described above. The box 130 provides a container for the reservoir 110, and as such, the box is not pressurized or sealed against an escape of liquid or air. The box may be provided with actuating buttons 138 on a top surface thereof. The box may also include feedback indicators 139 in the form of an array of LEDs. The feedback indicators 139 may indicate the amount of liquid emptied from the reservoir such that for example 5 indicators are used, and one light up when 1/5 has been emptied, the next light up when a further 1/5 has been emptied and so forth. The feedback may be obtained as explained below in relation to specific embodiments.

Figure 2 illustrates an embodiment of a reservoir 110, where the reservoir comprises two walls 111, 112, welded along three edges 113, 114, 115, each wall 111, 112 formed of a foil material. At the fourth edge, the reservoir comprises a proximal foil 116 inserted between the walls 111, 112. A tube 117 is attached to the top foil 116. In the illustrated embodiment, the tube 117 is firmly attached to the top foil, e.g., by welding the parts together such as shown at 118. The tube 117 may also be connected to a connector welded to the top foil, such as a snap-fit or bayonet connector. During use, the interior lumen of the reservoir is in fluid communication with an interior lumen of the tube, so that the irrigation liquid can flow from the reservoir and through the tube to an anal probe 119. In a proximal end of the tube, an anal probe is attached. In the illustrated embodiment, the anal probe 119 is firmly attached to the tube, but it may also be connected using a connector, e.g., a snap-fit or bayonet connector. In the illustrated embodiment the anal probe is shown with a closed tip 120 and an eyelet 121 through which irrigation liquid may exit during use. However, other configurations such as an open tip may also be used. The anal probe 119 may be provided with retention means (not shown) in the form of a foam cuff or an inflatable balloon.

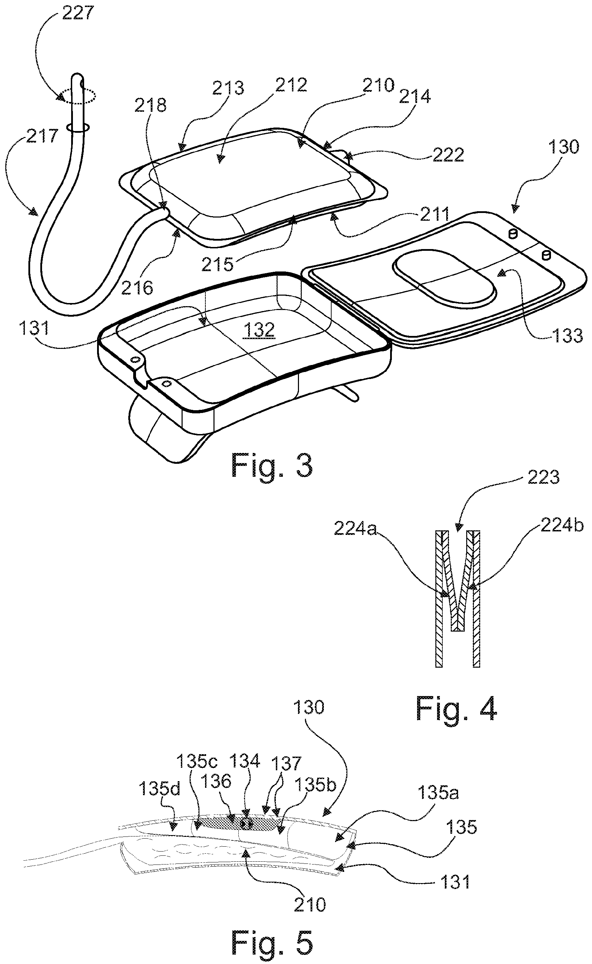

Figure 3 illustrates how the reservoir is configured to be held in the box 130. The reservoir 210 illustrated in figure 3 has walls 211, 212 which are welded along all four edges, 213, 214, 215, 216. At one weld, a filling spout 222 is provided. The tube 217 is welded to the reservoir in one of the welds as indicated at 218. The filling spout 222 may be in the form of one-way valve, with valve foils 224a, 224b inserted in the opening 223 as indicated in figure 4.

The box 130 has a cavity 131, which is configured for holding the reservoir 210 during the irrigation procedure. In the illustrated embodiment, the box 130 is made of rigid material and it is an advantage if the floor or lower interior wall 132 of the cavity is rigid. The floor 132 is the lowermost part of the cavity, when the box sits on the leg of a user. The floor 132 should be suitable for providing a back-stop for means for pressing 133 in the box. The means for pressing 133 is in the illustrated embodiment in the form of a pump 134 (see figure 5) and an inflatable lung 135, which is best seen in the cross-sectional view in figure 5. The inflatable lung may be subdivided into a number of chambers 135a, 135b, 135c, 135d, which allows for a feedback to the user of how big a portion of the reservoir has been subjected to pressure - and thereby how much liquid has left the reservoir. The box 130 includes a second cavity 136 in the lid, which is configured for holding the pump 134 and connections to the pump (not shown) from actuating buttons on a top surface - see figure 1. The pump may be provided with air-inlets 137 leading air into the second cavity 136.

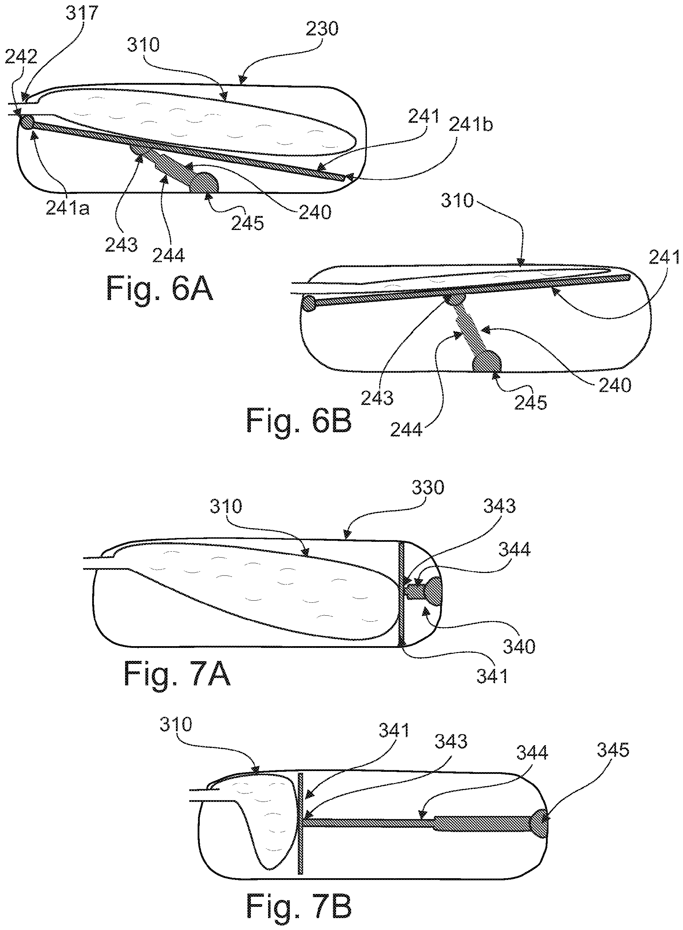

Figures 6A, 6B illustrate a box 230 and a reservoir 310 inserted into the box 230, where the means for pressing are in the form of an actuator 240 positioned under a tiltable pressure plate 241. The pressure plate 241 is tiltable around a setting 242, which provides for a hinged attachment of the pressure plate 241 to an interior side wall of the box 230. The pressure plate thus extends from a first end 241a attached to the setting 242 to a second end 241b which is freely movable up and down. The actuator 240 comprises a ball-shaped connection 245 at the lower side of the box, a linear actuator part 244, which is telescopically extendable and a support 243 for supporting the pressure plate 241. In a first position, as shown in figure 6A, the linear actuator part 244 is extended so that the support 243 is positioned closer to the first end 241a at the setting 242 than to the second end 241b of the floor.

In figure 6B, the pressure plate 241 is tilted around the setting 242. The actuator is in a more upright position with respect to the lower side of the box, such that linear actuator 244 is slightly shorter due to having been telescopically collapsed slightly as compared to figure 6A. At the same time, the actuator is rotated at the rotatable ball-shaped connection 245. This means that the support 243 has moved sideways toward the middle portion of the floor 241 and thus the pressure plate tilts from the upper right corner of the box downwards towards the setting 242. Thus, little room is left for the reservoir, which has then been squeezed. As a result, the liquid has been displaced from the reservoir through the tube 317 and out the anal probe (not shown). The position of the actuator can provide a feedback to the user of how much pressure has been put on the reservoir and thereby how much liquid has left the reservoir.

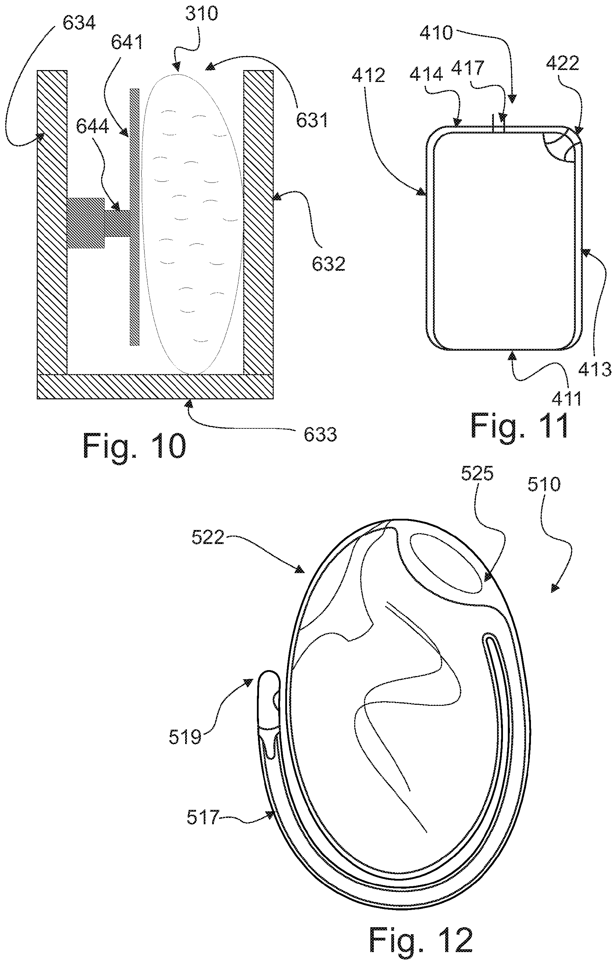

The reservoir 310 is illustrated in figure 11 as a folded foil, which is closed and welded at three sides 312, 313, 314 and folded at the fourth side 311. The tube 317 is welded to the top 313 of the reservoir e.g. by inserting the tube between the foils and welding around it. (. At the top, there is also a filling spout 322. The filling spout 322 is conveniently located at one corner of the reservoir and may be formed with a foil-valve as illustrated in figure 4.

Figures 7A, 7B illustrate a box 330 including a reservoir 310; the reservoir 310 is in this embodiment the same as in the embodiment of figures 6A, 6B. The box 330 comprises a linear actuator 340 and a pressure plate 341. The linear actuator 340 includes an actuator rod 344 which is attached to a sidewall of the box at a connection 345 and to the pressure plate at a connection 343. In figure 7A, the pressure plate 341 is at its rest position and the reservoir is still filled with irrigation liquid. The linear actuator 340 is also in its rest position with actuator rod 344 completely collapsed. In figure 7B, the pressure plate 341 is at a position closer to the opposite sidewall and the reservoir is nearly emptied. The linear actuator 340 is at an extended position with the actuator rod 344 telescopically extended compared to figure 7A. The extension of the actuator rod will allow for providing a feedback to user about how much pressure has been put on the reservoir and thereby how much liquid has left it.

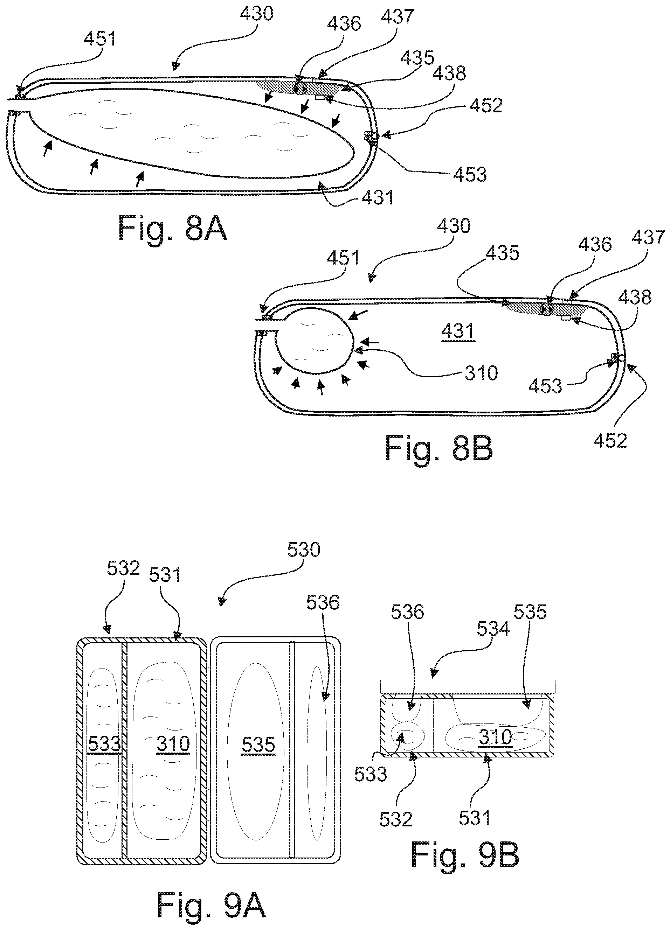

Figures 8A, 8B illustrate a box 430 having a pump 450 for increasing the pressure inside the box. The box 430 is sealed 451 at the tube connection, and also sealed 452 at the hinge 453 oppositely positioned to the tube connection. Furthermore, there are seals at the edges (not shown) thus providing an air-tight first cavity 431. When the pump 450 pumps air into the interior of the box, the pressure inside the box will increase and thus displace the liquid from the reservoir - indicated by the arrows. The pump 450 is fed air through the inlets 437 at a top of the box. The inlets may conveniently be provided with one-way valves so as to prevent air from leaving through these inlets. The pump 450 is located in a second cavity 436 at a top of the box. Air is fed from the pump through a connection 438 into the larger first cavity 431, which includes the reservoir 310. The amount of air pumped into the cavity 431 allow for a feedback to the user on how much pressure has been put on the reservoir and thereby how much liquid has left it.

Figures 9A and 9B illustrate a box 530, which has two chambers 531, 532 inside it. The larger chamber 531 is configured for holding the reservoir bag as described in the embodiments above. The smaller chamber 532 is configured for holding a smaller reservoir 533 of liquid, which is used to inflate an inflatable balloon on an anal probe (see figure 3). The lid of the box 534 has two inflatable lungs, a larger lung 535 configured for functioning as means for pressing on the reservoir for irrigation liquid and a smaller lung 536 configured for functioning as means for pressing on the smaller reservoir for inflating the balloon. A common pump (not shown) may be used to selectively inflate both lungs 535, 536 and may be electrically or manually driven. There may be a simple switch switching between pumping of the air between the two lungs.

Figure 10 illustrate a box 630, which is upstanding as compared to the embodiments illustrated in figure 6, 7, 8 and 9. This means that the sidewalls 632, 634 are larger than the bottom wall 633. In the illustrated embodiment, a linear actuator 640 as illustrated in connection with figures 7A, 7B, is used as means for pressing. The linear actuator has an actuator rod 644, which is shown halfway extended. The reservoir 310 is shown as halfway empty and being subjected to pressure from the pressure plate 641. This embodiment has the advantage that no lid is required. The gravity on the reservoir 310 will ensure that this is kept inside the cavity 635 of the box.

Figure 12 illustrates a reservoir 510, tube 517, catheter 519 all in one, which is made in a compact way. The reservoir 510 is generally elliptically shaped, and the tube 517 is cut as notch going approximately halfway around the ellipse. At a proximal end of the tube, an anal probe 519 is firmly attached. Furthermore, there may be a filling spout 522 in one end and a handle 525 conveniently positioned on the reservoir.

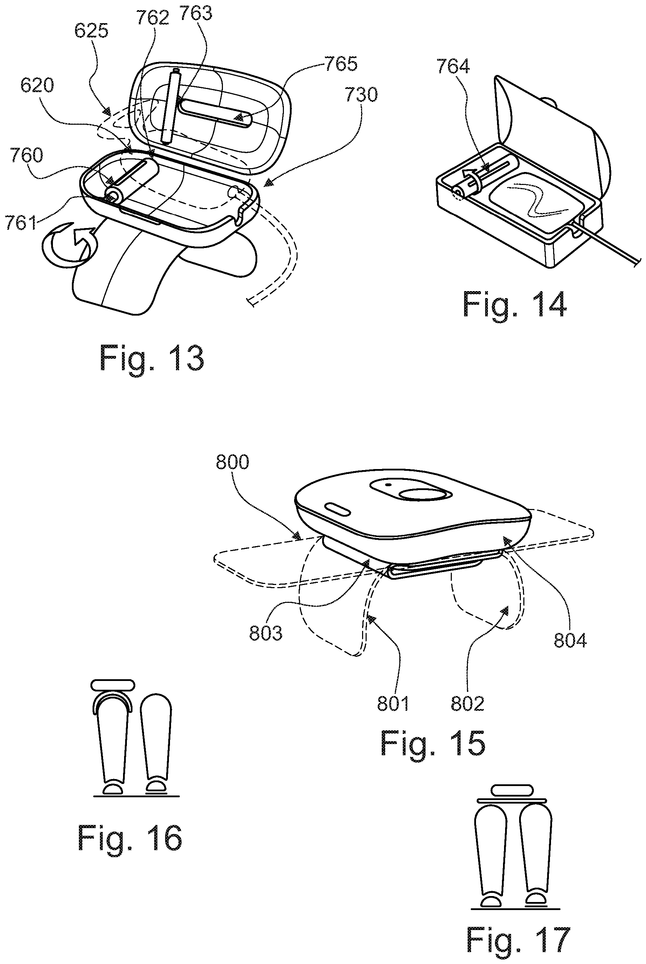

Figures 13 and 14 illustrate an anal irrigation system, where the box 730 includes means for pressing in the form of a roller 760. The roller is attached to sidewalls of the box at 761 and opposite to that at 762 so that the roller can roll inside the box. The box may further include a catch 763 for cooperating with the roller to catch the reservoir 620 between them. The reservoir 620 is provided with handles 625, which may be held during filling of the reservoir. An opening (not shown) into the reservoir may be positioned between the handles. The only difference between figure 13 and 14 is that instead of using a catch as in figure 13, the roller is provided with a slot 764 through which the reservoir can be inserted and caught prior to curling up around the roller. The box may be provided with a window 765 in a lid, so that the user can see how much of the reservoir bag has been curled up around the roller.

Figure 15-17 illustrates holding means 800 for a box, the box is in the figure the same box 130 as in figure 1, however any type of box as disclosed herein may be used. The holding means 800 consists of two arms 801, 802, which are foldable between three configurations. A first configuration, where the arms are folded in under the box - this is mostly convenient for storage purposes (figure 13). In a second configuration, the arms are folded downwards, such that they can be used to grab around a thigh of a user (figure 14). In a third configuration, the arms are folded outwards such that they provide a larger base for positioning the box across both thighs of the user (figure 15). In the illustrated embodiment, the arms 801, 802 comprises hinges 803, 804 allowing for 180 degrees movement of them.

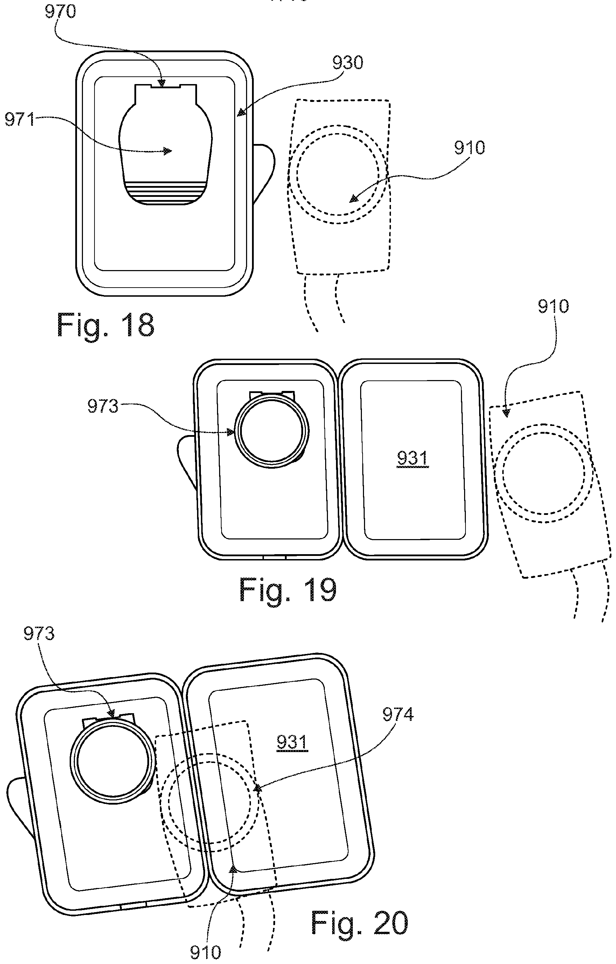

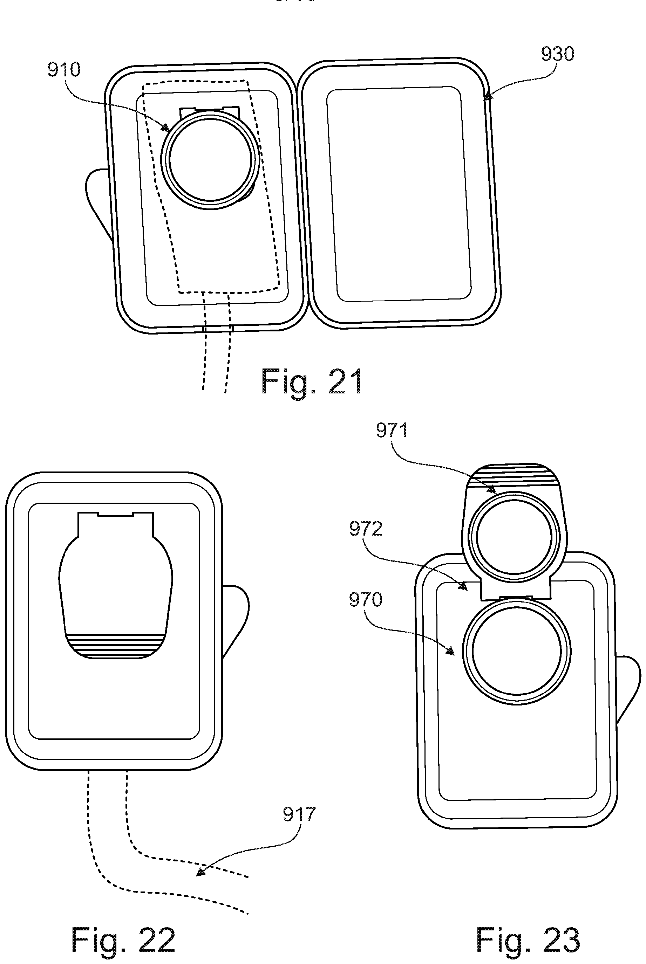

Figures 18 to 23 illustrate steps of preparing a box 930 and a reservoir 910 for use in a complete anal irrigation system. Figure 24 illustrates a cross-sectional view of the same box 930 and 910 when it is ready to use. The tubes 917 can be seen in figure 21. The box has a cavity 931 which is configured for holding the reservoir 910 during use. The means for pressing may be in the form of a pump 934 and an inflatable lung 935 (see figure 24). As described in relation to figure 5, the pump 934 will be able to suck in air through the air-inlets 937. The pump 934 is positioned in a cavity 936 in the lid. The box has a filling opening 970, which can be closed by a closure 971. In the illustrated embodiment, the closure 971 is attached to the box 930 at hinge 972 - however, the closure may be completely removable from the box. On the interior side of the filling opening 970, the box is provided with second coupling means 973 which is adapted for cooperating with first coupling means 974 on the reservoir bag 910. The first coupling means 974 can be seen in figure 20. In figure 21, the first and second coupling means are coupled together, such that the reservoir 910 is attached to the box 930 at the filling opening. In figure 22, the lid on the box is closed and the reservoir is now inside the cavity of the box. Figure 23 illustrates how the closure 971 can be opened so as to allow filling of the reservoir inside the box through the filling opening 970.

Figures 25-27 illustrate an embodiment not forming part of the invention as claimed, with a box 1030 which can be used without a reservoir. The box is closed and provided with a filling opening 1070, which can be closed by a closure 1071. Like with the box 930 in figures 18-24, this closure 1071 is attached to the box 1030 via a hinge 1072, however, the closure may be completely removable from the box. Liquid is filled into a cavity 1031 of the box through the filling opening 1070 and will flow around freely in this cavity 1031 (see figure 27). The means for pressing comprise a pump 1034 and an inflatable lung 1035, which functions as described above. The pump 1036 is positioned in a second cavity 1036 in the box 1030 and air is fed to the pump through air-inlets 1037 (see figure 27). The tubes 1017 are connected to the box by a luer-connector 1080 on the tube cooperating with a through-going opening 1081 on the box. Other types of connectors may be used, such as bayonet couplings or threaded connectors.

## Claims

1. An anal irrigation system (100) comprising
- an anal probe (119, 519), a reservoir (110, 210, 310, 510, 910) configured to contain irrigation liquid and tubes (117, 217, 317, 517, 917) connecting the reservoir (110, 210, 310, 510, 910) with the anal probe (119, 519), the reservoir (110, 210, 310, 510, 910) having flexible wall portions and
- a box (130, 230, 330, 430, 530, 630, 730, 930) having a shell that forms an exterior surface of the box and defines an interior space within the box, and the interior space of the box includes a reservoir holding cavity (131, 431, 531, 931), the box (130, 230, 330, 430, 530, 630, 730, 930) having means for pressing on an exterior surface of the reservoir thereby displacing the irrigation liquid from the reservoir (110, 210, 310, 510, 910), **characterised in that**
- the anal probe (119, 519) is firmly and not detachably connected to the tube (117, 217, 317, 517, 917), which in turn is firmly and not detachably connected to the reservoir (110, 210, 310, 510, 910).

2. The anal irrigation system (100) as in any of the preceding claims, wherein the means for pressing are positioned in a lid on the box.

3. The anal irrigation system (100) as in any of the preceding claims, wherein the means for pressing are positioned at a sidewall of the box.

4. The anal irrigation system (100) as in any of the preceding claims, wherein the means for pressing comprise a roller (760) configured for curling up the reservoir bag (110, 210, 310, 510, 910).

5. The anal irrigation system (100) as in claim 4, wherein the box (130, 230, 330, 430, 530, 630, 730, 930, 1030) comprises a fixture for fixating the reservoir (110, 210, 310, 510, 910) between the roller (760) and the fixture so as to keep the reservoir (110, 210, 310, 510, 910) fixated while it is curled up around the roller (760).

6. The anal irrigation system (100) as in claim 4, wherein the roller includes a slot (764) for inserting the reservoir (110, 210, 310, 510, 910) so as to keep the reservoir (110, 210, 310, 510, 910) fixated while the reservoir (110, 210, 310, 510, 910) is curled up around the roller (760).

7. The anal irrigation system (100) as in any of the preceding claims, wherein the means for pressing comprise an actuator (240, 340, 640) in combination with a pressure plate (241, 341, 641).

8. The anal irrigation system (100) as in claim 7, wherein the actuator (240, 340, 640) is a linear actuator (244, 340, 640) which is positioned at a sidewall of the box, the linear actuator (244, 340, 640) being configured for pushing the pressure plate (241, 341, 641) towards an opposite sidewall.

9. The anal irrigation system (100) as in claim 7, wherein the actuator (240, 340, 640) is rotatable and the pressure plate (241, 341, 641) is hingedly connected to a sidewall of the box and configured for being tilted with respect to the sidewall of the box.

10. The anal irrigation system (100) as in claim 9, wherein the actuator (240, 340, 640) is rotatable and linear.

11. The anal irrigation system (100) as in any of the preceding claims, wherein the means for pressing comprise an inflatable lung (135, 535, 536, 935) and an air pump (134, 450, 934).

12. The anal irrigation system (100) as in claim 11, wherein the inflatable lung (135, 535, 536, 935) is sub-divided into three, four or five chambers.

13. The anal irrigation system (100) as in claim 1, wherein the tube (117, 217, 317, 517, 917) and reservoir (110, 210, 310, 510, 910) are made from thin-walled material.

14. The anal irrigation system (100) as in any of the preceding claims, wherein the box (130, 230, 330, 430, 530, 630, 730, 930) comprises holding means (800) configured for holding the box (130, 230, 330, 430, 530, 630, 730, 930) in place at the upper side of the thighs of a user.

15. The anal irrigation system (100) as in claim 14, wherein the holding means (800) are in the form of a strap.

16. The anal irrigation system (100) as in claim 14, wherein the holding means (800) comprise a pair of foldable arms (801, 802) configured for, in an unfolded configuration, keeping the box (130, 230, 330, 430, 530, 630, 730, 930) positioned at a thigh of a user.

17. The anal irrigation system (100) as in any of the preceding claims, wherein the reservoir (110, 210, 310, 510, 910) is provided with first coupling means (974) and the box (130, 230, 330, 430, 530, 630, 730, 930) is provided with second coupling means (973), wherein the first coupling means (974) are configured for cooperating with the second coupling means (973) so as to provide a coupling for filling the reservoir (110, 210, 310, 510, 910) when the reservoir (110, 210, 310, 510, 910) is situated in the box (130, 230, 330, 430, 530, 630, 730, 930).

18. The anal irrigation system (100) as in claim 17, wherein the box (130, 230, 330, 430, 530, 630, 730, 930, 1030) 930) is provided with a filling opening (970) positioned at the coupling.

19. The anal irrigation system (100) as in any of claims 17 or 18, wherein the first coupling means (974) comprise a c-shaped channel configured for cooperating with a ridge on the second coupling means (973).

20. The anal irrigation system (100) as in any of claims 17 or 18, wherein the first coupling means (974) comprise a first upstanding ridge, which is configured for cooperating with a second upstanding ridge on the second coupling means (973), such that the first upstanding ridge circumferentially surrounds the second upstanding ridge.

21. The anal irrigation system (100) as in any of claims 17 to 20, wherein the box (130, 230, 330, 430, 530, 630, 730, 930) has a closure (971) for closing the filling opening (970).

## Patentansprüche

1. Analirrigationssystem (100), umfassend
- eine Analsonde (119, 519), ein Reservoir (110, 210, 310, 510, 910), das dazu ausgestaltet ist, Irrigationsflüssigkeit und Schläuche (117, 217, 317, 517, 917) zu enthalten, die das Reservoir (110, 210, 310, 510, 910) mit der Analsonde (119, 519) verbinden, wobei das Reservoir (110, 210, 310, 510, 910) flexible Wandabschnitte hat, und
- ein Kästchen (130, 230, 330, 430, 530, 630, 730, 930) mit einer Schale, die eine Außenfläche des Kästchens bildet und einen inneren Raum in dem Kästchen definiert, wobei der innere Raum des Kästchens einen Reservoirhaltehohlraum (131, 431, 531, 931) aufweist, wobei das Kästchen (130, 230, 330, 430, 530, 630, 730, 930) Mittel zum Drücken auf eine Außenfläche des Reservoirs hat, wodurch die Irrigationsflüssigkeit aus dem Reservoir (110, 210, 310, 510, 910) verdrängt wird, **dadurch gekennzeichnet, dass**
- die Analsonde (119, 519) fest und untrennbar mit dem Schlauch (117, 217, 317, 517, 917) verbunden ist, der seinerseits fest und untrennbar mit dem Reservoir (110, 210, 310, 510, 910) verbunden ist.

2. Analirrigationssystem (100) nach einem der vorhergehenden Ansprüche, wobei die Drückmittel in einem Deckel auf dem Kästchen positioniert sind.

3. Analirrigationssystem (100) nach einem der vorhergehenden Ansprüche, wobei die Drückmittel an einer Seitenwand des Kästchens positioniert sind.

4. Analirrigationssystem (100) nach einem der vorhergehenden Ansprüche, wobei die Drückmittel eine Rolle (760) umfassen, die zum Aufrollen des Reservoirbeutels (110, 210, 310, 510, 910) ausgestaltet ist.

5. Analirrigationssystem (100) nach Anspruch 4, wobei das Kästchen (130, 230, 330, 430, 530, 630, 730, 930, 1030) ein Befestigungsorgan zum Befestigen des Reservoirs (110, 210, 310, 510, 910) zwischen der Rolle (760) und dem Befestigungsorgan umfasst, um das Reservoir (110, 210, 310, 510, 910) festzuhalten, während es um die Rolle (760) gewickelt wird.

6. Analirrigationssystem (100) nach Anspruch 4, wobei die Rolle einen Schlitz (764) zum Einführen des Reservoirs (110, 210, 310, 510, 910) aufweist, um das Reservoir (110, 210, 310, 510, 910) festzuhalten, während das Reservoir (110, 210, 310, 510, 910) um die Rolle (760) aufgewickelt wird.

7. Analirrigationssystem (100) nach einem der vorhergehenden Ansprüche, wobei die Drückmittel einen Aktuator (240, 340, 640) in Kombination mit einer Druckplatte (241, 341, 641) umfassen.

8. Analirrigationssystem (100) nach Anspruch 7, wobei der Aktuator (240, 340, 640) ein Linearaktuator (244, 340, 640) ist, der an einer Seitenwand des Kästchens positioniert ist, wobei der Linearaktuator (244, 340, 640) dazu ausgestaltet ist, die Druckplatte (241, 341, 641) zu einer gegenüberliegenden Seitenwand hin zu schieben.

9. Analirrigationssystem (100) nach Anspruch 7, wobei der Aktuator (240, 340, 640) drehbar ist und die Druckplatte (241, 341, 641) scharniermäßig mit einer Seitenwand des Kästchens verbunden und zum Kippen bezüglich der Seitenwand des Kästchens ausgestaltet ist.

10. Analirrigationssystem (100) nach Anspruch 9, wobei der Aktuator (240, 340, 640) drehbar und linear ist.

11. Analirrigationssystem (100) nach einem der vorhergehenden Ansprüche, wobei die Drückmittel eine aufblähbare Lunge (135, 535, 536, 935) und eine Luftpumpe (134, 450, 934) umfassen.

12. Analirrigationssystem (100) nach Anspruch 11, wobei die aufblähbare Lunge (135, 535, 536, 935) in drei, vier oder fünf Kammern unterteilt ist.

13. Analirrigationssystem (100) nach Anspruch 1, wobei der Schlauch (117, 217, 317, 517, 917) und das Reservoir (110, 210, 310, 510, 910) aus einem dünnwandigen Material hergestellt sind.

14. Analirrigationssystem (100) nach einem der vorhergehenden Ansprüche, wobei das Kästchen (130, 230, 330, 430, 530, 630, 730, 930) Haltemittel (800) umfasst, die dazu ausgestaltet sind, das Kästchen (130, 230, 330, 430, 530, 630, 730, 930) an der Oberseite der Oberschenkel eines Benutzers zu halten.

15. Analirrigationssystem (100) nach Anspruch 14, wobei die Haltemittel (800) in der Form eines Gurts vorliegen.

16. Analirrigationssystem (100) nach Anspruch 14, wobei die Haltemittel (800) ein Paar Klapparme (801, 802) umfassen, die dazu ausgestaltet sind, in einer ausgeklappten Konfiguration das Kästchen (130, 230, 330, 430, 530, 630, 730, 930) an einem Oberschenkel eines Benutzers zu halten.

17. Analirrigationssystem (100) nach einem der vorhergehenden Ansprüche, wobei das Reservoir (110, 210, 310, 510, 910) mit ersten Koppelmitteln (974) versehen ist und das Kästchen 230, 330, 430, 530, 630, 730, 930) mit zweiten Koppelmitteln (973) versehen ist, wobei die ersten Koppelmittel (974) zum Zusammenwirken mit dem zweiten Koppelmittel (973) ausgestaltet sind, um eine Koppelung zum Füllen des Reservoirs (110, 210, 310, 510, 910) bereitzustellen, wenn das Reservoir (110, 210, 310, 510, 910) in dem Kästchen (130, 230, 330, 430, 530, 630, 730, 930) -angeordnet ist.

18. Analirrigationssystem (100) nach Anspruch 17, wobei das Kästchen (130, 230, 330, 430, 530, 630, 730, 930, 1030) 930) mit einer Füllöffnung (970) versehen ist, die an der Koppelung positioniert ist.

19. Analirrigationssystem (100) nach einem der Ansprüche nach einem der Ansprüche 17 oder 18, wobei die ersten Koppelmittel (974) einen c-förmigen Kanal umfassen, der zum Zusammenwirken mit einer Erhöhung an den zweiten Koppelmitteln (973) ausgestaltet ist.

20. Analirrigationssystem (100) nach einem der Ansprüche 17 oder 18, wobei die ersten Koppelmittel (974) eine erste aufrechtstehende Erhöhung umfassen, die zum Zusammenwirken mit einer zweiten aufrechtstehenden Erhöhung an den zweiten Koppelmitteln (973) ausgestaltet ist, so dass die erste aufrechtstehende Erhöhung die zweite aufrechtstehende Erhöhung umfangsmäßig umgibt.

21. Analirrigationssystem (100) nach einem der Ansprüche 17 bis 20, wobei das Kästchen (130, 230, 330, 430, 530, 630, 730, 930) einen Verschluss (971) zum Verschließen der Füllöffnung (970) aufweist.

## Revendications

1. Système d'irrigation anale (100) comprenant
- une sonde anale (119, 519), un réservoir (110, 210, 310, 510, 910) configuré pour contenir un liquide d'irrigation et des tubes (117, 217, 317, 517, 917) reliant le réservoir (110, 210, 310, 510, 910) à la sonde anale (119, 519), le réservoir (110, 210, 310, 510, 910) ayant des parties de paroi flexibles et
- un boîtier (130, 230, 330, 430, 530, 630, 730, 930) ayant une coque qui forme une surface extérieure du boîtier et définit un espace intérieur à l'intérieur du boîtier, et l'espace intérieur du boîtier comprend une cavité de maintien de réservoir (131, 431, 531, 931), le boîtier (130, 230, 330, 430, 530, 630, 730, 930) ayant des moyens de pression sur une surface extérieure du réservoir, déplaçant ainsi le liquide d'irrigation depuis le réservoir (110, 210, 310, 510, 910), **caractérisé en ce que**
- la sonde anale (119, 519) est reliée solidement et de manière non détachable au tube (117, 217, 317, 517, 917), qui est lui-même relié solidement et de manière non détachable au réservoir (110, 210, 310, 510, 910).

2. Système d'irrigation anale (100) selon l'une quelconque des revendications précédentes, dans lequel les moyens de pression sont positionnés dans un couvercle sur le boîtier.

3. Système d'irrigation anale (100) selon l'une quelconque des revendications précédentes, dans lequel les moyens de pression sont positionnés au niveau d'une paroi latérale du boîtier.

4. Système d'irrigation anale (100) selon l'une quelconque des revendications précédentes, dans lequel les moyens de pression comprennent un rouleau (760) configuré pour enrouler la poche de réservoir (110, 210, 310, 510, 910).

5. Système d'irrigation anale (100) selon la revendication 4, dans lequel le boîtier (130, 230, 330, 430, 530, 630, 730, 930, 1030) comprend une fixation pour fixer le réservoir (110, 210, 310, 510, 910) entre le rouleau (760) et la fixation de manière à maintenir le réservoir (110, 210, 310, 510, 910) fixé pendant qu'il est enroulé autour du rouleau (760).

6. Système d'irrigation anale (100) selon la revendication 4, dans lequel le rouleau comprend une fente (764) pour insérer le réservoir (110, 210, 310, 510, 910) de manière à maintenir le réservoir (110, 210, 310, 510, 910) fixé pendant que le réservoir (110, 210, 310, 510, 910) est enroulé autour du rouleau (760).

7. Système d'irrigation anale (100) selon l'une quelconque des revendications précédentes, dans lequel les moyens de pression comprennent un actionneur (240, 340, 640) en combinaison avec une plaque de pression (241, 341, 641).

8. Système d'irrigation anale (100) selon la revendication 7, dans lequel l'actionneur (240, 340, 640) est un actionneur linéaire (244, 340, 640) qui est positionné au niveau d'une paroi latérale du boîtier, l'actionneur linéaire (244, 340, 640) étant configuré pour pousser la plaque de pression (241, 341, 641) vers une paroi latérale opposée.

9. Système d'irrigation anale (100) selon la revendication 7, dans lequel l'actionneur (240, 340, 640) est rotatif et la plaque de pression (241, 341, 641) est reliée par charnière à une paroi latérale du boîtier et configurée pour être inclinée par rapport à la paroi latérale du boîtier.

10. Système d'irrigation anale (100) selon la revendication 9, dans lequel l'actionneur (240, 340, 640) est rotatif et linéaire.

11. Système d'irrigation anale (100) selon l'une quelconque des revendications précédentes, dans lequel les moyens de pression comprennent un poumon gonflable (135, 535, 536, 935) et une pompe à air (134, 450, 934).

12. Système d'irrigation anale (100) selon la revendication 11, dans lequel le poumon gonflable (135, 535, 536, 935) est subdivisé en trois, quatre ou cinq chambres.

13. Système d'irrigation anale (100) selon la revendication 1, dans lequel le tube (117, 217, 317, 517, 917) et le réservoir (110, 210, 310, 510, 910) sont réalisés en matériau à paroi mince.

14. Système d'irrigation anale (100) selon l'une quelconque des revendications précédentes, dans lequel le boîtier (130, 230, 330, 430, 530, 630, 730, 930) comprend des moyens de maintien (800) configurés pour maintenir le boîtier (130, 230, 330, 430, 530, 630, 730, 930) en place au niveau du côté supérieur des cuisses d'un utilisateur.

15. Système d'irrigation anale (100) selon la revendication 14, dans lequel les moyens de maintien (800) se présentent sous la forme d'une sangle.

16. Système d'irrigation anale (100) selon la revendication 14, dans lequel les moyens de maintien (800) comprennent une paire de bras pliables (801, 802) configurés pour, dans une configuration dépliée, maintenir le boîtier (130, 230, 330, 430, 530, 630, 730, 930) positionné au niveau d'une cuisse d'un utilisateur.

17. Système d'irrigation anale (100) selon l'une quelconque des revendications précédentes, dans lequel le réservoir (110, 210, 310, 510, 910) est pourvu de premiers moyens d'accouplement (974) et le boîtier (130, 230, 330, 430, 530, 630, 730, 930) est pourvu de seconds moyens d'accouplement (973), dans lequel les premiers moyens d'accouplement (974) sont configurés pour coopérer avec les seconds moyens d'accouplement (973) de manière à fournir un accouplement pour le remplissage du réservoir (110, 210, 310, 510, 910) lorsque le réservoir (110, 210, 310, 510, 910) est situé dans le boîtier (130, 230, 330, 430, 530, 630, 730, 930).

18. Système d'irrigation anale (100) selon la revendication 17, dans lequel le boîtier (130, 230, 330, 430, 530, 630, 730, 930, 1030) 930) est pourvu d'une ouverture de remplissage (970) positionnée au niveau de l'accouplement.

19. Système d'irrigation anale (100) selon l'une quelconque des revendications 17 ou 18, dans lequel les premiers moyens d'accouplement (974) comprennent un canal en forme de c configuré pour coopérer avec une arête sur les seconds moyens d'accouplement (973).

20. Système d'irrigation anale (100) selon l'une quelconque des revendications 17 ou 18, dans lequel les premiers moyens d'accouplement (974) comprennent une première arête dressée, qui est configurée pour coopérer avec une seconde arête dressée sur les seconds moyens d'accouplement (973), de telle sorte que la première arête dressée entoure circonférentiellement la seconde arête dressée.

21. Système d'irrigation anale (100) selon l'une quelconque des revendications 17 à 20, dans lequel le boîtier (130, 230, 330, 430, 530, 630, 730, 930) comporte une fermeture (971) pour fermer l'ouverture de remplissage (970).
